# EUROPEAN PATENT APPLICATION

(11) **EP 3 530 317 A1**
(43) Date of publication of application: **28.08.2019**
(21) Application number: 18196729.0
(22) Date of filing: 26.09.2018
(51) Int. Cl.: A61N 5/10

(54) **SYSTEM FOR IRRADIATING A PATIENT'S TUMOR**

(30) Priority: 22.02.2018 US 201815902011
(71) Applicant: Landeskrankenanstalten-Betriebsgesellschaft - KABEG, 9020 Klagenfurt am Wörthersee (AT)
(72) Inventor: TUBIN, Slavisa, 9020 Klagenfurt am Wörthersee (AT)
(74) Representative: Weiser & Voith Patentanwälte Partnerschaft

(57) **Abstract**

The disclosed subject-matter relates to a system (1) for treatment of a patient (2) by irradiating a tumor (3), the system (1) comprising: a scanner (105) configured to scan at least a part of a body (4) of the patient (2); a processor (106) being configured to localize the tumor (3) in the scan (S') and to determine a hypoxic and a normoxic region (H, N) of the tumor (3) and a micro-environment (M) surrounding the tumor (3); an irradiation device (109) configured to emit irradiation beams (12, 12') in different irradiation directions (dᵢ) that intersect in the hypoxic region (H); and a controller (108) configured to control the irradiation device (109) such that an accumulated irradiation dose delivered to the hypoxic region (H) during the treatment is greater than 8 Gy and an accumulated irradiation dose delivered to the micro-environment (M) during the treatment is, in an average over said micro-environment, less than 3 Gy.

## Description

### TECHNICAL FIELD

The disclosed subject-matter relates to a system for treatment of an oncological patient by irradiating a tumor by means of an irradiation device.

### BACKGROUND ART

Traditional radiobiology is based on the theory that the effects of radiotherapy present themselves only within an irradiated tissue of a patient, through direct and indirect DNA damage (Cook A.M., Berry R.J., Direct and indirect effects of irradiation: their relation to growth, Nature, 1966, 210:324-325) .

Currently, after sporadic reports that stated that irradiation effects occasionally occur outside the irradiated tissue, the local effects were denominated as targeted effects, and the distant ones as non-targeted effects (Nagasawa H., Little J.B., Induction of sister chromatid exchanges by extremely low irradiation doses of alpha-particles, Cancer Res, 1992, 52:6394-6396) .

Two types of non-targeted effects were described, depending on the site of their occurrence and the relationship between the irradiated and non-irradiated tumor:
- The Radiation-Induced Abscopal Effect (RIAE) is a systemic effect of local irradiation, which means it extends to distant non-irradiated tissues outside the treated tissue. Basically, this means that tumor regression is observed at distant untreated sites.
- The Radiation-Induced Bystander Effect (RIBE) is a radiobiological effect-transmission that happens when irradiation of only a part of the tumor induces regression of the whole tumor.

However, both phenomena have only been sporadically clinically observed, as they were occasional and unintentional and it was not possible to induce RIAE/RIBE on a consistent basis.

### BRIEF SUMMARY

It is an object of the disclosed subject-matter to provide an effective system for treatment of a patient, for example metastatic patients with large (bulky) tumors, by irradiating a tumor.

To this end, in a first aspect the disclosed subject-matter provides for a system for treatment of a patient by irradiating a tumor, the system comprising:
a scanner configured to scan at least a part of a body of the patient to generate a scan, said part comprising the tumor and surrounding tissue;
a processor connected to the scanner for receiving said scan, the processor being configured to localize the tumor in the scan and to determine a hypoxic and a normoxic region of the tumor and a micro-environment of tissue surrounding the tumor in the scan;
an irradiation device configured to emit irradiation beams in at least two different irradiation directions that intersect in the hypoxic region of the tumor; and
a controller connected to the processor and to the irradiation device, the controller being configured to control the irradiation device with regard to the irradiation directions and to an individual irradiation dose in each irradiation direction such that an accumulated irradiation dose delivered to the hypoxic region during the treatment is greater than 8 Gy and an accumulated irradiation dose delivered to the micro-environment during the treatment is, in an average over said micro-environment, less than 3 Gy.

For the control and eradication of a tumor, conventional systems of radiotherapy cover the entire tumor with the prescribed ionizing irradiation dose. To assure that all cells within the tumor will indeed be irradiated and potentially be killed, an additional margin around the visible tumor needs to be applied during the treatment planning to create a so called "CTV" (Clinical Target Volume) including both the tumor and high-risk tissue surrounding the tumor ("peritumoral" tissue) including possible microscopic tumor infiltration outside the detectable tumor. The size of this margin varies, but is usually in the range of about 1 cm. For compensation of an eventual setup-error during the patient's positioning and tumor movements, e.g., during respiration, a further margin is applied around the CTV, thusly creating a "PTV" (Planning Target Volume) which includes the CTV; the size of this further margin greatly varies but is usually again about 1 cm. With this conventional approach, which is the standard of care, the PTV containing the tumor micro-environment is irradiated by conventional systems with full radiotherapy dose as homogeneously as possible (at least 95%) whereby the tumor micro-environment receives the same radiation dose as the whole tumor.

In contrast thereto, when targeting the ionizing irradiation dose to the hypoxic region of the tumor with only little loss to the normoxic region, it has been experienced that RIAE/RIBE occurs fairly consistently when the peripheral normoxic tumor region and healthy tissues are not primarily subjected to irradiation. While not being targeted during the irradiation treatment, both the normoxic tumor region and healthy tissues are, however, exposed to irradiation during the treatment when irradiating the inside hypoxic region.

In order to understand the underlying mechanisms and to improve the effectiveness of inducing the non-targeted effects, latest research concentrated not only on the hypoxic region but also on the micro-environment of the tumor. In a clinical study it has been found that it was possible to induce non-targeted effects of radiotherapy in a very high percentage of the study population by not only irradiating the hypoxic region with high-dose radiotherapy and thereby delivering an accumulated irradiation dose of more than 8 Gy but, at the same time, controlling the accumulated irradiation dose delivered to the micro-environment of the tumor to a level below 3 Gy during the treatment. The response rates for RIBE and RIAE were at surprising 97% and 52.5%, respectively, being absolutely the highest rates ever reported.

The effectiveness of the present system consists in inducing and exploiting the existing natural anti-tumor immunity and "self-destructive" (the opposite to the metastatic process) abscopal tumor signaling, boosting those available anti-tumor mechanisms. Since the main mediators of the non-targeted effects of radiotherapy are infiltrating peritumoral immune cells and signaling molecules, it is essential to protect these carriers of anti-tumor immunity by preserving the tumor micro-environment from irradiation. To preserve the micro-environment of the tumor, the spatial average of the accumulated irradiation dose delivered to the micro-environment is controlled to be kept below the specified level. When in some spatial regions of the micro-environment the accumulated irradiation dose exceeds said level, e.g., due to the shape and size of the tumor and the hypoxic region therein, the accumulated irradiation dose in other spatial regions of the micro-environment is controlled to be sufficiently below said level in order to protect more carriers of anti-tumor immunity in the latter regions of the micro-environment.

It shall be noted that the required accumulated irradiation dose to be delivered to the hypoxic region depends primarily on the type of tumor and is known in the art. Moreover, the accumulated irradiation dose delivered to the normoxic region of the tumor is not necessarily controlled by the present system; the normoxic region is rather utilized as a buffer to achieve a sufficient decline of accumulated irradiation dose from the hypoxic region to the micro-environment.

While the accumulated irradiation doses delivered to the hypoxic region and to the micro-environment, respectively, during the treatment depend, inter alia, on the size and shape of the tumor and on irradiation treatment planning by the controller, it is preferred that the controller is configured to control the irradiation device such that said accumulated irradiation dose delivered to the micro-environment during the treatment is, in an average over said micro-environment, less than 2 Gy for obtaining even higher protection of the carriers of anti-tumor immunity in the micro-environment of the tumor.

In an advantageous embodiment, the system further comprises a support on which the irradiation device is supported movably about the body of the patient, wherein the controller is further configured to control the moving of the irradiation device on the support. Thereby, the system effectuates the irradiation treatment from different locations about the body of the patient; the system can even deliver the irradiation doses uninterruptedly, when the irradiation device is moved continuously during the emission of irradiation beams.

In a further favorable embodiment, the controller is configured to control the irradiation device to emit the irradiation beams in a predetermined number of irradiation directions such that segments of the micro-environment remain un-irradiated between said irradiation beams, which segments cover at least 50% of the micro-environment. The segments of the micro-environment left un-irradiated in this embodiment preserve a favorably high quantity of carriers of anti-tumor immunity. It shall, however, be noted that due to inevitable side-effects of the treatment a minor irradiation dose is also delivered to those segments of the tumor micro-environment which are, according to this principle, un-irradiated; this irradiation dose is, however, typically well below 1 Gy.

Preferably, said predetermined number of irradiation directions is two to four for obtaining un-irradiated segments covering a high percentage of the micro-environment.

The irradiation device can be chosen depending on the available setup for irradiation, for example an irradiation device for stereotactic radiation therapy (SBRT) or three-dimensional conformal radiotherapy (3D-CRT) with multi-leaf-collimator, intensity modulated radiation therapy (IMRT), volumetric modulated arc therapy (VMAT), or brachytherapy, and/or with a proton, electron, or heavy charged particle beam. These irradiation devices can be classified in two main categories, which will be outlined in the following.

In the first category of irradiation devices, the irradiation device comprises an aperture, e.g., a multi-leaf collimator, wherein the controller is further configured to set the aperture of the irradiation device for each irradiation direction separately to match a contour of the hypoxic region of the tumor as seen in that irradiation direction and to control the irradiation device to emit the irradiation beams in that irradiation direction through the aperture such that the aperture blocks the irradiation beams from irradiating the normoxic region and the micro-environment during the treatment.

This embodiment has the advantage that irradiation devices can be utilized that emit irradiation beams of larger diameter. Such irradiation devices usually have one irradiation source emitting a single or more irradiation beams, each of which having a diameter that is larger than a typical hypoxic region of a tumor, in one irradiation direction; the irradiation source can be moved and rotated about the patient's body, optionally having the tumor or the hypoxic region of the tumor, respectively, as a center for rotation.

In the second category of irradiation devices, the irradiation device is configured to emit the irradiation beams in different irradiation directions that intersect at a single movable irradiation spot, wherein the controller is further configured to define a plurality of different target points inside the hypoxic region and no target points outside the hypoxic region and to move the irradiation spot onto each of the defined target points such that the irradiation spot is neither moved to the normoxic region nor to the micro-environment during the treatment.

This embodiment has the advantage that irradiation devices can be utilized that emit one or more irradiation beams having a diameter smaller, usually at least a tenth smaller, than a typical hypoxic region of a tumor. Between the irradiation beams, segments of the micro-environment remain un-irradiated. The irradiation device of this type can have multiple irradiation sources about the patient's body emitting the irradiation beams in different irradiation directions, or again only a single (or few) irradiation source(s) that rotate(s) about the patient for generating the irradiation beams in different irradiation directions.

In an advantageous embodiment, the scanner is configured to use a positron emission tomography to generate the scan. Hence, the tumor and the hypoxic and normoxic regions as well as the micro-environment surrounding the tumor can readily be identified. To this end, the processor is preferably configured to calculate a standardized uptake value of the positron emission tomography scan and to determine the hypoxic region as a region in which the standardized uptake value, e.g., in a case of using 18F-FDG as a tracer, is equal to or smaller than 3. The region with the standard uptake value of 3 or less usually corresponds to an unvascularized or hypovascularized part of the tumor, which correlates to the hypoxic region.

Depending on the embodiment and tumor type, the scanner may be configured to use a tracer 18F-FMISO or 18F-FDG for positron emission tomography. Tracer 18F-FMISO is a hypoxia-specific tracer, meaning that a hypoxic region can be detected with a high degree of certainty, and is thus well suited for the system. Furthermore, tracer 18F-FDG having an acceptable predictive value for tumor hypoxia can be used especially among some aggressive tumor types like in those patients with gastric carcinoma, tongue cancer, non-small cell lung cancer (NSCLC), or oral squamous cell carcinoma.

In another embodiment, the scanner is configured to use a computed tomography, which is optionally contrast enhanced, to generate the scan by combining a result of the positron emission tomography with a result of the computed tomography. Thereby, the scanner generates particularly high quality scans.

### BRIEF DESCRIPTION OF THE DRAWINGS/FIGURES

The disclosed subject-matter shall now be explained in more detail below on the basis of exemplary embodiments thereof with reference to the accompanying drawings, in which:
Fig. 1 shows a system for irradiation treatment of a patient in a block diagram,
Figs. 2a to 2c show, in a first embodiment of the system of Fig. 1, a first (Fig. 2a) and a second step (Fig. 2b) of the treatment in a schematic cross-section, and an aperture of an irradiation device of the system (Fig. 2c) in a plan view,
Figs. 3a, 3b show, in a second embodiment of the system of Fig. 1, a first (Fig. 3a) and a second step (Fig. 3b) of the treatment in a schematic cross-section,
Figs. 4a - 4e show different cross-sections of a patient's thoracic cavity with a voluminous squamous cell carcinoma in the right lung (Fig. 4a), distant mediastinal lymphadenopathies/metastases (Fig. 4b), an induction of a bystander effect by targeting the hypoxic region (Fig. 4c), great reduction of whole partially treated tumor due to a bystander effect-induction after three weeks (Fig. 4d), and disappearance of untreated distant mediastinal lymphadenopathies due to an abscopal effect (Fig. 4e),
Fig. 5 shows a variation of the system of Fig. 1 for a different type of irradiation treatment of a patient in a block diagram,
Figs. 6a to 6d show, in a first embodiment of the system of Fig. 5, a first (Fig. 6a) and a second step (Fig. 6b) of the treatment, an overview of the resulting irradiation during the treatment (Fig. 6c) in respective schematic cross-sections, and an aperture of an irradiation device of the system (Fig. 6d) in a plan view,
Figs. 7a, 7b show, in a second embodiment of the system of Fig. 5, a first (Fig. 7a) and a second step (Fig. 7b) of the treatment in a schematic cross-section, and
Figs. 8a - 8d show different cross-sections of a patient's thoracic cavity with a voluminous squamous cell carcinoma in the right lung (Fig. 8a), a lower lobe lung lesion (Fig. 8c), great reduction of the whole partially treated tumor due to a bystander effect-induction after three weeks (Fig. 8b) and of the untreated distant lower lobe lung lesion due to an abscopal effect (Fig. 8d).

### DETAILED DESCRIPTION

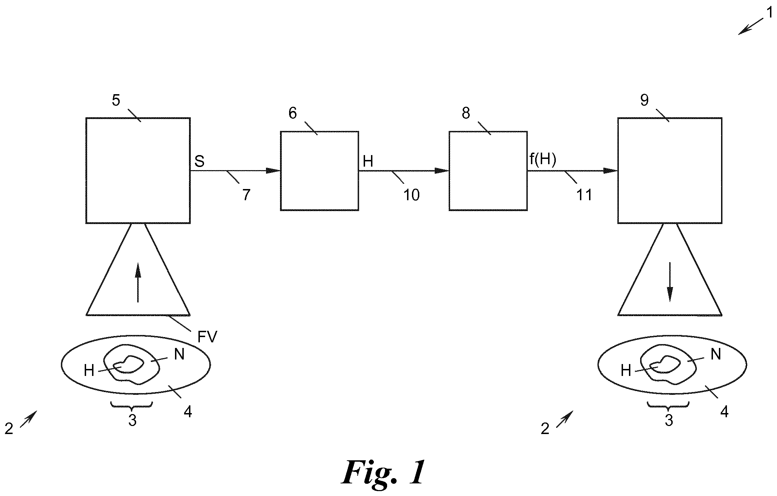

Fig. 1 shows a system 1 for treatment of a patient 2 by irradiating a tumor 3, wherein only a cross-section of the patient's body 4 is depicted in Fig. 1. As known in the state of the art, tumors 3 of the type discussed herein have a hypoxic region H and a normoxic region N. The hypoxic region H is a region that usually comprises a hypovascularized and hypometabolic tumor segment, while the normoxic region N has a hypermetabolic tumor segment. In such tumors 3, the hypoxic region H is commonly surrounded by the normoxic region N.

For localizing the tumor 3 within the patient's body 4, the system comprises a scanner 5, which scans at least a part of the body 4 of the patient 2 to generate a scan S. The generated scan S is a three-dimensional (3D) scan such that a 3D model of the part of the body 4 and of the tumor 3 can be generated. Depending on the embodiment, the 3D scan S can also be composed of a multitude of two-dimensional (2D) scans.

The scan S can be generated by any method known in the art, for example by positron emission tomography or computed tomography. Said methods can also be combined to obtain a higher-quality scan S, for example by combining a result of the positron emission tomography with a result of the computed tomography. For positron emission tomography, a tracer can be used, for example tracer 18F-FMISO or 18F-FDG, which are especially suited for detecting the hypoxic region H as outlined below. It shall be understood that a different tracer may be used as known in the art.

The scan S can be a scan of the whole body 4 of the patient but also only a partial scan S of the body 4, i.e., the part of the body 4 comprising the tumor 3. For this purpose, the scanner 5 can have an adjustable field of view FV which can either be set mechanically or computationally.

The scanner 5 is connected to a processor 6 for receiving the scan S by means of a connection 7, for example a wired or wireless (WiFi, Bluetooth, RFID, et cet.) direct connection or an indirect connection such as a USB storage medium for the scan S that can be connected to interfaces on both the scanner 5 and processor 6.

After receiving the scan S, the processor 6 localizes the tumor 3 therein and determines the hypoxic region H and the normoxic region N of the tumor 3 in the scan S, again as 3D regions. This can be done by means of any method known in the art, for example by measuring a standardized uptake value (SUV) of the scan S created by positron emission tomography with the abovementioned tracers. A SUV of, e.g., 3 or less may indicate the hypoxic region H while a standard uptake value larger than 3 within the tumor would consequently indicate the normoxic region N.

The system 1 further comprises a controller 8 and an irradiation device 9. The controller 8 is connected to the processor 6 by means of a connection 10 that can be of the types as the abovementioned connection 7. The controller 8 and the processor 6 can be separate from each other or alternatively be integrated in one and the same physical entity or even be implemented as different tasks/applications in one and the same processing device. In another embodiment, the controller 8 is a part of the irradiation device 9.

The controller 8 controls the irradiation device 9 via a connection 11 that can also be of any type mentioned above with respect to the connection 7. The controller 8 has the purpose of interacting with actuators or drive components of the irradiation device 9 to adjust the irradiation device 9 to target only the hypoxic region H determined by the processor 6, whereof the controller 8 received the information on the hypoxic region H for controlling the irradiation device 9. This is symbolically shown as control function f(H) of the controller 8.

Thereafter, the irradiation device 9 irradiates the targeted hypoxic region H.

The system 1 only targets the hypoxic region H for irradiation. During the whole treatment, which can take several minutes (usually not more than 20 minutes), depending, inter alia, on the irradiation device 9, the normoxic region N is not targeted for irradiation.

During the treatment, the irradiation device 9 delivers an irradiation dose of, e.g., at least 8 Gy for irradiation of the hypoxic region H, i.e., in a single fraction.

The irradiation device 9 can be of any type known in the state of the art, for example it can be operated by means of three-dimensional conformal radiotherapy (3D-CRT) with multi-leaf collimator, intensity modulated radiation therapy (IMRT), volumetric modulated arc therapy (VMAT), or a photon, electron, or heavy charged particle beam. Different ways of irradiating are disclosed in US 8,395,131 B2, for example, which is hereby incorporated by reference.

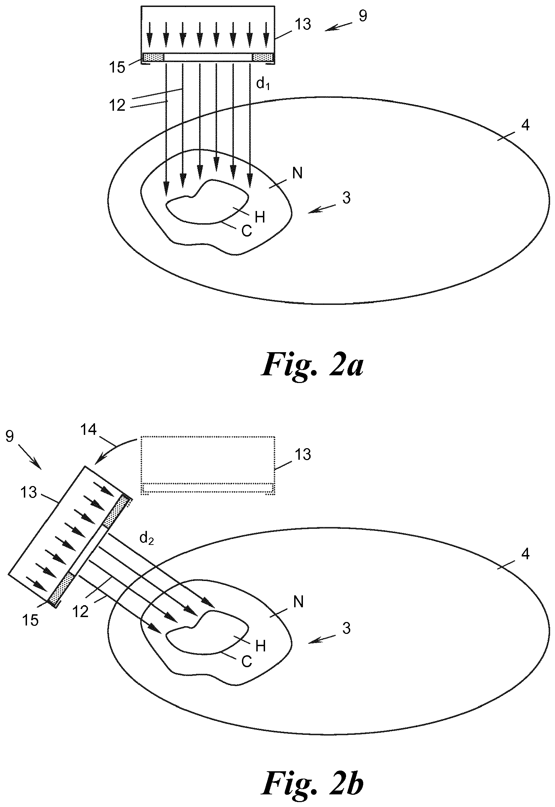

Figs. 2a and 2b show a type of irradiation device 9 that emits irradiation beams 12 in different irradiation directions d₁, d₂, ..., generally dᵢ, that intersect at the hypoxic region H. Specifically, in Fig. 2a the irradiation device 9 emits irradiation beams 12 in a first irradiation direction d₁ onto the hypoxic region H, while in Fig. 2b the irradiation device 9 emits irradiation beams 12 in a second irradiation direction d₂ onto the hypoxic region H after the irradiation device 9, or at least its head 13, was rotated about the body 4 of the patient 2 (arrow 14) .

To ensure that only the hypoxic region H and not the normoxic region N is targeted throughout the treatment, the irradiation device 9 comprises an aperture 15, which can for example be embodied as a collimator. The targeting is performed by setting the aperture 15 of the irradiation device 9 to match a contour C of the hypoxic region H of the tumor 3 as seen in the irradiation direction dᵢ. To this end, the opening of the aperture 15 is variable.

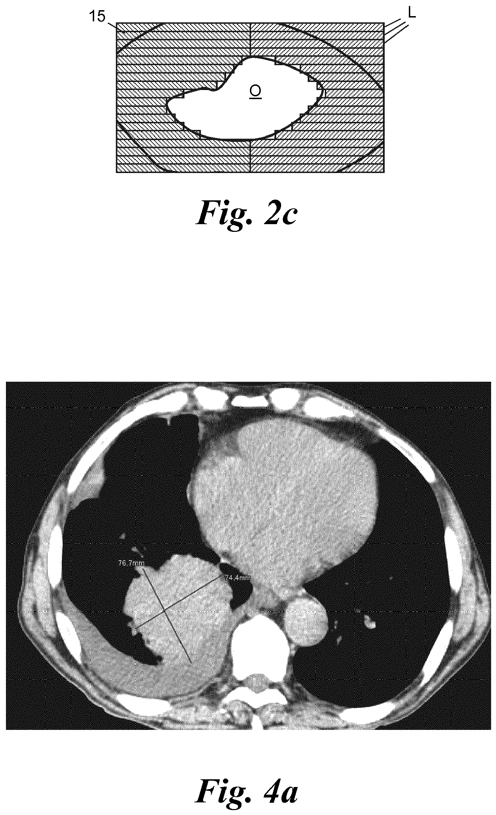

Fig. 2c shows an example of such an aperture 15. The aperture 15 of this example comprises longitudinal cover strips L that can be inserted into and retracted from the aperture 15 from both sides to variably create an opening O that matches the contour C of the hypoxic region H as seen in the irradiation direction dᵢ. An alternative could be an aperture 15 that is embodied as holes that are arranged in an array, whereupon holes can be selectively covered such that the opening O matches the shape of the contour C.

After the aperture 15 has been set to match the contour C of the hypoxic region H, the irradiation device 9 performs the step of irradiating by emitting irradiation beams 12 in the first irradiation direction d₁ through the aperture 15 onto the targeted hypoxic region H. The aperture 15 thus blocks irradiation beams 12 from irradiating the normoxic region N outside said contour C.

After irradiating in the first irradiation direction d₁, the irradiation device 9 (or its head 13) rotates so that it can irradiate the hypoxic region H from the second irradiation direction d₂ which is different from the first irradiation direction d₁.

When necessary for the treatment of the patient 2, the irradiation device 9 continues to irradiate the hypoxic region H in further irradiation directions d₃, d₄, ..., dᵢ, ... until it will deliver a full planned irradiation dose to the hypoxic region. The controller 8 and the irradiation device 9 are configured to perform the targeting and irradiating for each respective irradiation direction dᵢ of the treatment as described above.

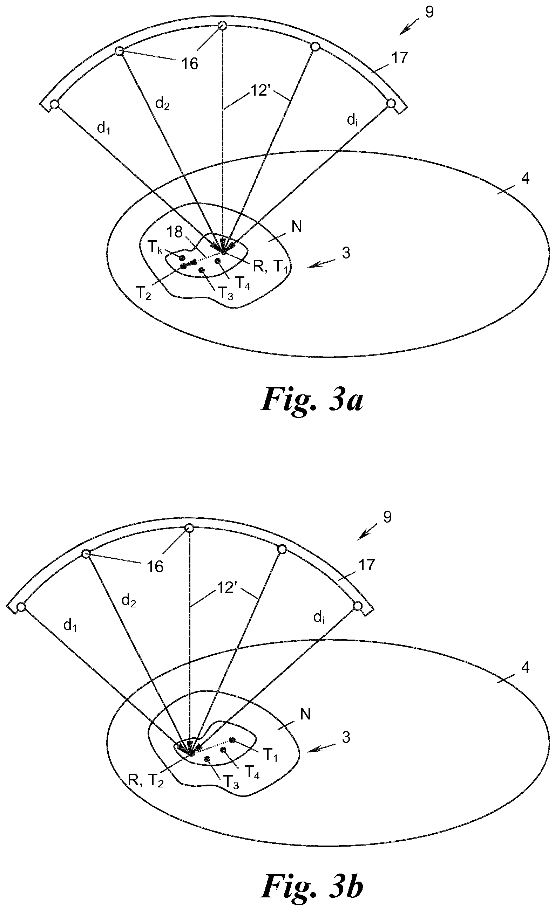

Fig. 3a shows an exemplary embodiment of a different type of irradiation device 9. In the shown embodiment, the irradiation device 9 consists of a multitude of irradiation sources 16 that are fixated on a mounting 17. The individual irradiation sources 16 each emit an irradiation beam 12' in a respective different irradiation direction d₁, d₂, ..., dᵢ, ..., such that the irradiation beams 12' intersect at a single movable irradiation spot R. The irradiation beams 12' each have a cross section that is smaller than a typical diameter of the hypoxic region H to be treated, e.g., 10 times smaller. Hence, also the irradiation spot R is smaller than the typical diameter of the hypoxic region H.

Alternatively to the multiple irradiation sources 16 on the mounting 17, again a single irradiation source 16 could be used that rotates about the irradiation spot R such that each of the emitted irradiation beams 12' intersect at an irradiation spot R.

For such an irradiation device 9 using irradiation beams 12' of a small cross section, the controller 8 defines, before targeting, a plurality of different target points T₁, T₂, ..., Tₖ, ..., T_{K}, within the hypoxic region H. No target points Tₖ are defined in the normoxic region N during the treatment to exclusively target the hypoxic region H. Typically, the target points Tₖ are defined such that they have a predefined density in the hypoxic region H, e.g., 1 target point Tₖ per cm³.

For targeting the hypoxic region H, the controller 8 "moves" the irradiation spot R onto the first target point T₁, i.e., selects the first target point T₁ for irradiation, as shown in Fig. 3a, whereupon the irradiation device 9 emits said irradiation beams 12 in all of said different irradiation directions dᵢ onto said first target point T₁.

The moving of the irradiation spot R (selecting of the target spot Tₖ) can be performed by applying a rotational or translational movement to the irradiation device 9 or the mounting 17 of the irradiation sources 16 as controlled by the controller 8. In an alternative embodiment, the irradiation sources 16 can individually be tilted with respect to the mounting 17 such that the irradiation spot R can be moved by said tilting.

After the first target point T₁ has been selected and irradiated, the second target point T₂ is selected, i.e., the irradiation spot R is moved to the second target point T₂ that is different from the first target point T₁ (dotted arrow 18), and is then irradiated. This is repeated for each of said plurality of different target points Tₖ defined in the hypoxic region H.

When irradiating the tumor 3 as outlined above, attention should be focused on the tumor type, its radio-sensitivity, irradiation dose and fractionation schedule, tumor volume to be targeted, and stressing tumor hypoxia. There is evidence supporting the hypothesis that RIBE and RIAE are mediated by the immune-system that probably is involved, but it is not the dominant component that determines the manifestation of those phenomena, otherwise, every single metastatic patient exposed to immunotherapy (that enhances the effects of the immune system) in addition to radiotherapy would manifest it, which is mostly not the case. According to the inventor's studies, non-targeted effects were induced in most cases, but none of the treated patients used immunotherapy. Considering how many patients are treated every day with radio-immunotherapy without manifesting non-targeted effects shows that this phenomenon is still rare despite the fact that all patients are immune-competent. Therefore, by establishing suitable conditions considering the previously mentioned factors, it is possible to intentionally induce RIAE/RIBE, as will be exemplified by cases 1 and 2 further down.

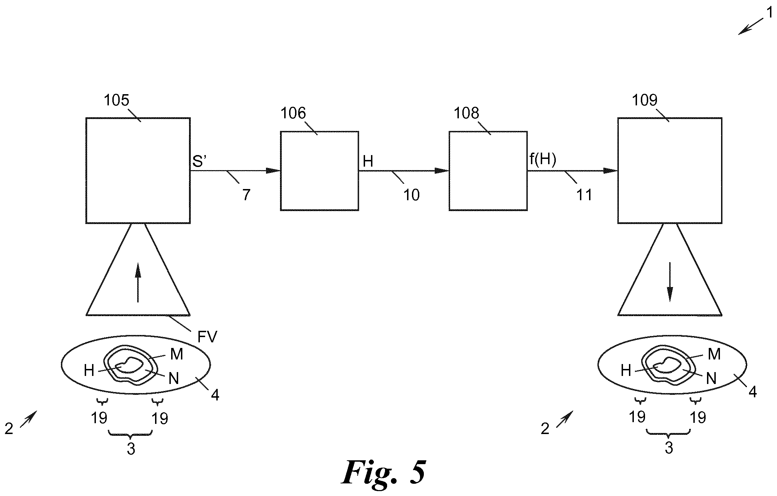

In the following, a variation of the system 1 of Figs. 1 to 3, but for a different type of treatment of the patient 2, shall be explained with reference to Figs. 5 to 7.

To avoid redundancies, in Figs. 5 to 7 same elements as in the system 1 of Figs. 1 to 3 are designated with the same references signs. An element in Figs. 5 to 7 which is similar to an element in Figs. 1 to 3 is designated with a reference numeral that is higher by 100 than the reference numeral of the respective element of Figs. 1 to 3; for such similar elements, only those details that functionally or structurally differ from the details of the respective elements of Figs. 1 to 3 are explained below, whereas all other details are the same as described before.

The system 1 of Figs. 5 to 7 comprises a scanner 105 which scans, in one of the ways described above with respect to Fig. 1, at least a part of the patient's body 4, the part comprising the tumor 3 and surrounding ("peritumoral") tissue 19, to generate a 3D scan S'. The scanner 105 is connected to a processor 106 via a connection 7, as described above. In the scan S' received from the scanner 105, the processor 106 localizes the tumor 3 and determines the hypoxic region H and the normoxic region N therein as well as a micro-environment M surrounding the tumor 3 in the scan S', e.g., by measuring a standardized uptake value (SUV) of the scan S'.

The system 1 further comprises a controller 108 and an irradiation device 109. The controller 108 is connected to processor 106 via a connection 10 and to the irradiation device 109 via a connection 11, as described above, respectively. The controller 108 and the processor 106 can be separate from each other or, alternatively, be integrated in one and the same physical device, or even be implemented as different tasks or applications in one and the same processing device. Furthermore, the controller 108 may optionally be a part of the irradiation device 109.

The irradiation device 109 emits irradiation beams 12 in at least two different irradiation directions dᵢ (i = 1, 2, ...) that intersect in the hypoxic region H of the tumor 3. Moreover, the irradiation device 109 can be of any type as described above in relation to the irradiation device 9 of Figs. 1 to 3.

As mentioned above, the controller 108 controls the irradiation device 109 with regard to the irradiation directions dᵢ and to an individual irradiation dose delivered in each irradiation direction dᵢ. The controlling of the irradiation device 109 includes treatment planning by the controller 108 prior to emission of irradiation beams 12 by the irradiation device 109. Treatment planning may be performed as a task or application of the controller 108 or by a separate processing device of the controller 108, which processing device is optionally located in a different physical entity and connected, e.g., as described above for connection 7, to the remaining portion of the controller 108.

The individual irradiation dose delivered in each irradiation direction dᵢ can be individually controlled, inter alia depending on the irradiation device 109, e.g., by means of the duration of emitting irradiation beams 12 in that irradiation direction dᵢ, the intensity of irradiation in that direction dᵢ, and/or the type of irradiation (e.g., proton, electron or heavy ion particle irradiation) in that direction dᵢ.

The controller 108 controls the irradiation device 109 such that an accumulated irradiation dose delivered to the hypoxic region H during the treatment is greater than 8 Gy, and that an accumulated irradiation dose delivered to the micro-environment M during the treatment is less than 3 Gy, or even less than 2 Gy. The term "accumulated" refers to an accumulation of the individual irradiation doses delivered in all different irradiation directions dᵢ during the treatment. Said accumulated irradiation dose delivered to the micro-environment M is to be understood as a spatial average, i.e. an average over the volume of the micro-environment M.

The micro-environment M is part of the peritumoral tissue 19. More precisely, the micro-environment M is mostly located in a margin around the visible tumor which creates a volume known in prior art treatments as "CTV" (Clinical Target Volume) and is partly located in a further margin around the CTV which creates a volume known in prior art treatments as "PTV" (Planning Target Volume). The margins of both the CTV and the PTV are chosen according to the individual case and are typically each in the range of about 1 cm.

In the embodiment of the system 1 of Fig. 1, solely the hypoxic region H is targeted for irradiation, i.e., when controlling the irradiation device the irradiation dose of the hypoxic region H is controlled while merely keeping irradiation dose delivered to the body 4 of the patient 2 low but without specifically considering the normoxic region N or the micro-environment M of the tumor 3. In contrast thereto, in the variation according to Figs. 5 to 7, the controller 108 now controls both the accumulated irradiation dose delivered to the hypoxic region H (to be greater than 8 Gy during the treatment) and the accumulated irradiation dose delivered to the micro-environment M of the tumor 3 (to be less than, e.g. , 3 Gy, in an average over the micro-environment M, during the treatment).

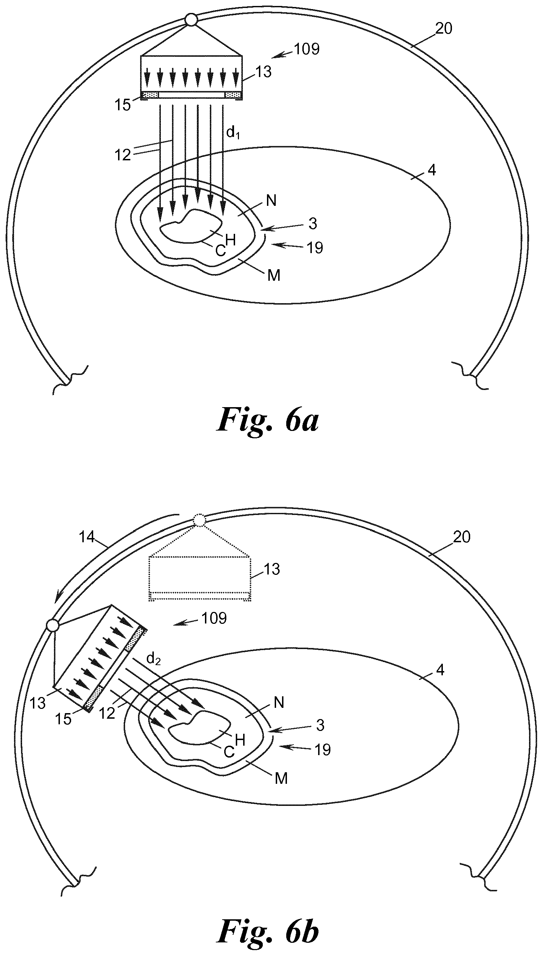

In a first embodiment according to Figs. 6a and 6b, the system 1 comprises a support 20. The irradiation device 109, or at least its head 13, is supported movably about the patient's body 4 on the support 20. The support 20 is, e.g., ring-, tube- or arc-shaped for receiving the patient's body 4 inside. By moving the irradiation device 109 on the support 20, the irradiation device 109 is also rotated about the body 4 (arrow 14). The support 20 is connected to the controller 108 which controls the moving of the irradiation device 109 (or its head 13) on the support 20, e.g., from a first position shown in Fig. 6a (and in dotted lines in Fig. 6b), where the irradiation device 109 emits irradiation beams 12 in a first direction d₁, to a second position shown in Fig. 6b, where the irradiation device 109 emits irradiation beams 12 in a second direction d₂ which is different from the first direction d₁ and intersects the first direction d₁ in the hypoxic region H of the tumor 3.

Alternatively, the irradiation device 109 may be fixated to an end of an electrically or hydraulically driven arm which itself is movable and controlled by the controller 108 such that the irradiation device 109 is moved and rotated about at least a part of the patient's body 4.

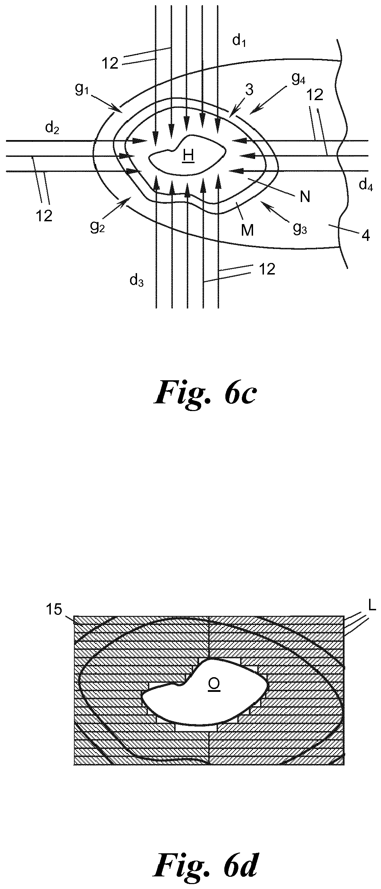

The irradiation device 109 may, in an embodiment or during one treatment, emit irradiation beams 12 continuously while also being moved practically continuously during the treatment. On the other hand, as shown in the example of Fig. 6c, the irradiation device 109 may be moved from one position to another, while only emitting irradiation beams 12 when stopped in one of said positions. In this case, the controller 108 controls the irradiation device 109 to emit irradiation beams 12 in a predetermined number of irradiation directions dᵢ, e.g., in two, three or, as shown in this example, four irradiation directions d₁, d₂, d₃ and d₄, which intersect in the hypoxic region H of the tumor 3.

In the embodiment of Fig. 6c, the irradiation directions dᵢ are chosen such that segments g₁, g₂, ..., generally gⱼ, of the micro-environment M remain un-irradiated between the irradiation beams 12; the remainder of the micro-environment M, however, is irradiated by the irradiation beams 12 which are emitted to or pass through the hypoxic region H in the respective irradiation directions dᵢ. It shall be noted that, while being un-irradiated, there is still a minor irradiation dose delivered to said segments gⱼ due to the nature of irradiation and depending on the type of the irradiation device 109; this irradiation dose is, however, usually well below 1 Gy.

In one embodiment, the un-irradiated segments gⱼ cover at least 50% of the volume of the micro-environment M. Thereby, the accumulated irradiation dose delivered to the micro-environment M during the treatment is, in a spatial average over said micro-environment M, less than 3 Gy (or even less than 2 Gy), while an accumulated irradiation dose delivered to the hypoxic region H during the treatment of greater than 8 Gy can be achieved. This effectuates the protection of carriers of anti-tumor immunity located in the micro-environment M.

As shown in the example of Fig. 6d, the irradiation device 109 optionally comprises an aperture 15, e.g., a collimator, with longitudinal cover strips L that variably create an opening O. The controller 108 sets the aperture 15 for each irradiation direction dᵢ of the irradiation device 109 separately such that, e.g., the opening O of the aperture 15 matches the contour C of the hypoxic region H of the tumor 3 as seen in that irradiation direction dᵢ, i.e. the aperture 15 (or its opening O) matches the contour C of the hypoxic region H as seen in the first irradiation direction d₁ when emitting irradiation beams 12 in that first irradiation direction d₁ (Fig. 6a), and matches the contour C of the hypoxic region H as seen in the second irradiation direction d₂ when emitting irradiation beams 12 in that second irradiation direction d₂ (Fig. 6b), etc. The irradiation device 109 is controlled by the controller 108 to emit the irradiation beams 12 in the respective irradiation direction dᵢ, i.e. in the example of Fig. 6a in the first irradiation direction d₁ and in the example of Fig. 6b in the second irradiation direction d₂, through the aperture 15 such that the aperture 15 blocks the irradiation beams 12 from irradiating the normoxic region N and the micro-environment M during the treatment.

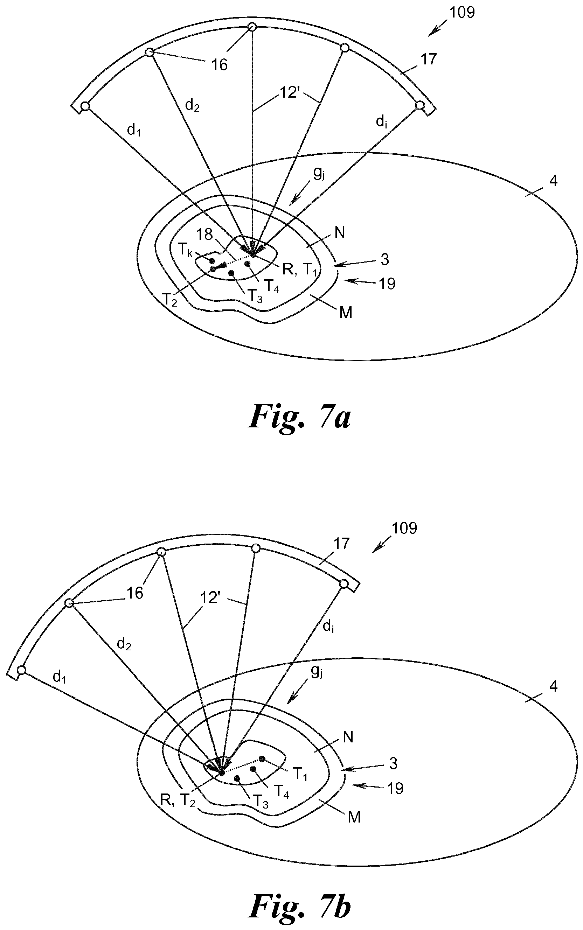

Figs. 7a and 7b show an embodiment of the system 1 using a different type of irradiation device 109 which is similar to the irradiation device 9 described in detail above relating to Figs. 3a and 3b. Also the irradiation device 109 of the embodiment of Figs. 7a and 7b comprises a multitude of irradiation sources 16, each of which is fixated on the mounting 17 and emits an irradiation beam 12' in a respective different irradiation direction dᵢ. The irradiation beams 12' thusly emitted in different irradiation directions dᵢ by the irradiation device 109 intersect at a single movable irradiation spot R which is smaller than the typical hypoxic region H.

The controller 108 defines a plurality of different target points Tₖ (k = 1, 2, ...) inside the hypoxic region H and no target points Tₖ outside the hypoxic region H. During the treatment, the controller 108 moves (dotted arrow 18) the irradiation spot R onto each of the defined target points Tₖ such that the irradiation spot R is neither moved to the normoxic region N nor to the micro-environment M. Thereby, the controller 108 can effectuate that the accumulated irradiation dose delivered to the hypoxic region H during the treatment is greater than 8 Gy and that the accumulated irradiation dose delivered to the micro-environment M during the treatment is, in an average over said micro-environment M, less than 3 Gy (or even less than 2 Gy), e.g., by leaving segments gⱼ between the irradiation beams 12' un-irradiated. In this way, the carriers of anti-tumor immunity located in the micro-environment M are protected.

In this embodiment, the mounting 17 can partly surround the body 4 of the patient 2 with multiple irradiation sources 16 fixated to the mounting 17, as in the example of Figs. 7a and 7b. The mounting 17 can optionally be movable and/or rotatable about the body 4 during the treatment. In this case, only a few or even a single irradiation source 16 is/are sufficient for the irradiation device 109. In an alternative embodiment, the mounting 17 (almost) completely surrounds the patient's body 4, i.e., the patient 2 lies inside the mounting 17, with a greater multitude of irradiation sources 16 distributed on and fixated to the mounting 17, which in this embodiment may be immovable.

### EXAMPLES

The abovementioned system has been tested to prove the validity of this new strategy in terms of the induction of RIAE/RIBE in the tumor. Specifically, a first small group of highly selected patients was treated by targeting exclusively the hypoxic region of their bulky tumors with high-dose radiotherapy as previously described with respect to Figs. 1 to 3.

The evaluation of response was performed when significant regression of symptoms was recorded (average 2 - 3 weeks). The Response Evaluation Criteria in Solid Tumors (RECIST) were used for that purpose. The average volume of the targeted hypoxic region (mean: 65.6 cm³, range: 42.6 - 90.2 cm³) represented about only 30% of whole bulky tumor (mean: 212.9 cm³, range: 132.5 - 298.2 cm³; mean diameter: 7.9 cm, range: 7 - 10 cm) . The average maximum SUV (SUVₘₐₓ) of bulky tumors was 19.3 (range: 15.2 - 26.7), while it was only 2.7 in the hypoxic region (range: 1 - 3), which represented 15% of SUVₘₐₓ among the treated tumor.

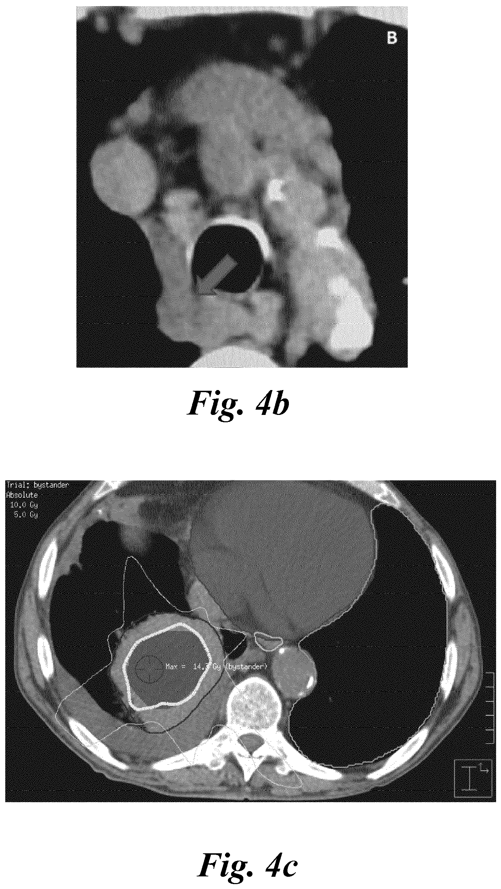

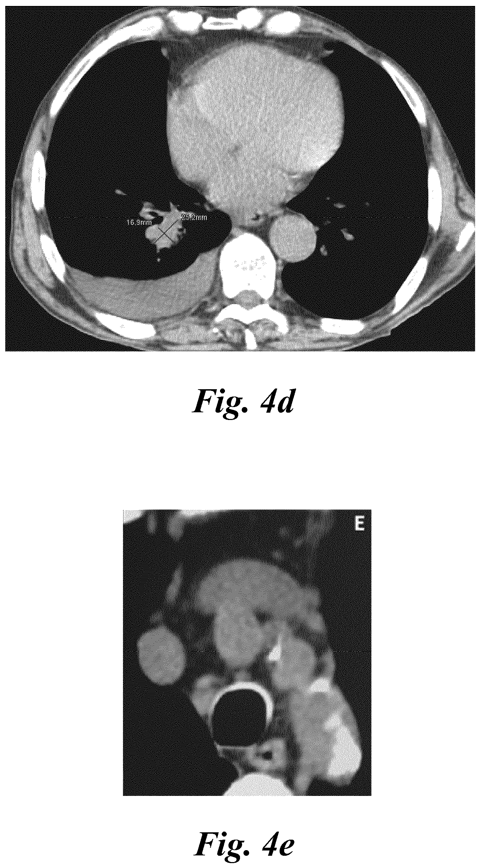

Among all patients, a significant RIBE was observed, with an intense tumor regression after an average of three weeks. Additionally, a significant RIAE was observed in about 50 % of cases (Figs. 4b and 4e). Overall, the response rate for the relief of the symptoms and mass reduction was 100%. The symptoms, for which the patients underwent radiotherapy, were under control at their last follow-up and the irradiated tumors did not re-grow. The maximum bulky downsizing (regression) was achieved after an average of four weeks with an average tumor shrinkage of 60 %. No patient experienced any acute or late toxicity of any grade. No patient was treated with systemic therapy immediately prior, during, or immediately after radiotherapy so that the effects observed belong only to the radiation treatment presented herein.

A second group of highly selected patients was treated by controlling the accumulated irradiation dose delivered to the micro-environment M to be less than 3 Gy while the accumulated irradiation dose delivered to the hypoxic region H of their bulky tumors was greater than 8 Gy during the treatment as described above with reference to Figs. 5 to 7.

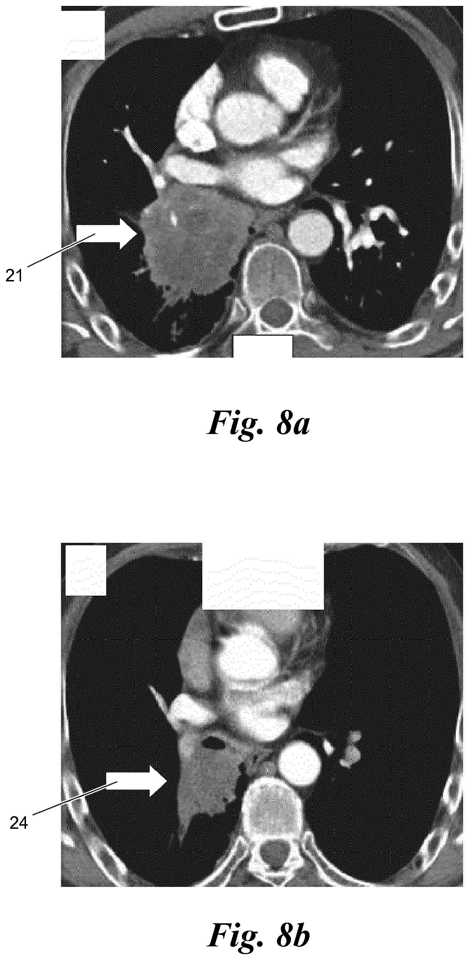

The following three cases best exemplify the feasibility and efficacy of the disclosed subject-matter, wherein cases 1 and 2 relate to the variation exemplified by Figs. 1 to 3 and case 3 (Figs. 8a to 8d) relates to the variation exemplified by Figs. 5 to 7.

### Case 1:

A 78-year-old male with a history of prostate cancer (which had been treated with radiotherapy eight years earlier), an adenocarcinoma of the ascending colon (for which he underwent a right hemicolectomy and adjuvant chemotherapy one year earlier), now presented with a voluminous left neck metastasis, from a squamous cell carcinoma of the right ear that was previously resected in combination with a modified radical neck dissection. The 9.7 cm large tumor was un-resectable due to its infiltration of the cervical vertebra bodies. Chemotherapy was not an option. Due to increasing pain, he was treated with radiotherapy, 10 Gy in a single fraction to the 70% isodose-line (14 Gy) to the centrally located hypoxic region, corresponding to only 30 % of the whole bulky tumor. Only two weeks later, about 50 % of tumor regression was observed, which started from the tumor's non-irradiated periphery towards the irradiated center. The patient had no longer any pain.

### Case 2:

A 73-year-old male, previously resected for a malignant melanoma and with a history of hypothyroidism and nicotine/alcohol abuse, was diagnosed with a right lower-lung lobe squamous cell carcinoma. His tumor was very voluminous, causing dyspnea and pain. At that time, the carcinoma measured 10.9 cm (Fig. 4a). Furthermore, there were also mediastinal lymphadenopathies that, because asymptomatic, were not subjected to treatment (Fig. 4b), and two small metastases in the contralateral lung. Because of a poor performance status, both resection and systemic therapy were unsuitable. Due to the important symptoms, the multidisciplinary tumor board recommended palliative irradiation of the lung tumor. Also for this patient we chose a single 10 Gy fraction to the 70% isodose-line, prescribed to the hypoxic tumor segment (Fig. 4c) . Four weeks later, a dramatic regression was observed; the tumor had reduced to 2 cm (Fig. 4d), and the dyspnea and pain completely disappeared. Furthermore, the untreated mediastinal lymphadenopathies also disappeared due the RIAE (Fig. 4e).

### Case 3:

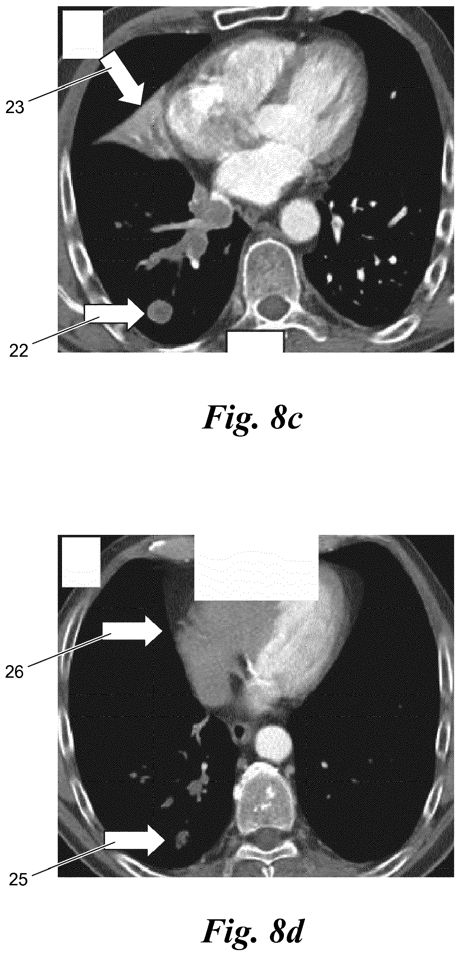

A 59-year-old male presented with a histologically proven, central, un-resectable 8 cm large bulky squamous-cell lung cancer (arrow 21 in Fig. 8a). In addition, he had a separate 2 cm large lower lobe lung lesion (arrow 22 FIG. 8c), a tumor-induced atelectasis (arrow 23 in FIG. 8c), and metastatic mediastinal lymph nodes: tumor stage cT4(m) cN2 cM0, IIIB. The patient received irradiation exclusively to his primary un-resectable bulky lung cancer: 10Gy x 3 to 70%-isodose line (Dmax 43.5 Gy) prescribed to the hypoxic region while keeping the dose delivered to the micro-environment at less than 3 Gy; the other two tumor sites (Fig. 8c) were not irradiated and the patient did not get any systemic treatment, neither chemotherapy nor immunotherapy.

Only 3 weeks after irradiation, a preoperative CT scan showed 60%, 50% and 30% reductions of partially irradiated bulky (arrow 24 in Fig. 8b), un-irradiated separate lung lesion (arrow 25 in Fig. 8d) and metastatic lymph nodes, respectively, and a complete regression of the atelectasis (arrow 26 in FIG. 8d), so that the remaining tumor was defined resectable and right bilobectomy with lymph node dissection was performed.

Histological evaluation showed a massive 80%-necrosis in the bulky tumor with a dense immune reaction at the border of the necrosis by infiltration of lymphocytes (regression grade IIA sec. Junker in Junker K., Therapy-induced tumor regression and regression grading in lung cancer, Pathologe, 2014; 35:574-7). Also, in a separate lung lesion, which was another primary lung cancer but adenocarcinoma, a massive 80%-central necrosis has been recorded (IIA sec. Junker). Three un-irradiated mediastinal metastatic lymph nodes were completely necrotic (regression grade III sec. Junker); the tumor stage after the treatment was resulted retro-staged: ypT2a ypN0 (0/15) cM0, stage yIB.

The immunohistochemistry, performed on the available tissue samples after surgery showed that the apoptosis-inducing factor (AIF), responsible for a significant necrosis at all three tumor sites, was massively upregulated in the partially irradiated bulky tumor, but also in the un-irradiated lung adenocarcinoma and mediastinal lymph node metastases. This appears to indicate a significant induction of tumor cells-killing, also at un-treated distant tumor sites as a result of the immune system-activation and abscopal tumor signaling.

The disclosed subject-matter is not restricted to the specific embodiments described in detail herein, but encompasses all variants, combinations and modifications thereof that fall within the framework of the appended claims.

## Claims

1. A system for treatment of a patient (2) by irradiating a tumor (3), the system (1) comprising:
a scanner (105) configured to scan at least a part of a body (4) of the patient (2) to generate a scan (S'), said part comprising the tumor (3) and surrounding tissue (19);
a processor (106) connected to the scanner (105) for receiving said scan (S'), the processor (106) being configured to localize the tumor (3) in the scan (S') and to determine a hypoxic and a normoxic region (H, N) of the tumor (3) and a micro-environment (M) surrounding the tumor (3) in the scan (S');
an irradiation device (109) configured to emit irradiation beams (12, 12') in at least two different irradiation directions (dᵢ) that intersect in the hypoxic region (H) of the tumor (3); and
a controller (108) connected to the processor (106) and to the irradiation device (109), the controller (108) being configured to control the irradiation device (109) with regard to the irradiation directions (dᵢ) and to an individual irradiation dose in each irradiation direction (dᵢ) such that an accumulated irradiation dose delivered to the hypoxic region (H) during the treatment is greater than 8 Gy and an accumulated irradiation dose delivered to the micro-environment (M) during the treatment is, in an average over said micro-environment, less than 3 Gy.

2. The system according to claim 1, wherein the controller (108) is configured to control the irradiation device (109) such that said accumulated irradiation dose delivered to the micro-environment (M) during the treatment is, in an average over said micro-environment, less than 2 Gy.

3. The system according to claim 1 or 2, wherein the system (1) further comprises a support (20) on which the irradiation device (109) is supported movably about the body (4) of the patient (2), and wherein the controller (108) is further configured to control the moving of the irradiation device (109) on the support (20).

4. The system according to any one of claims 1 to 3, wherein the controller (108) is configured to control the irradiation device (109) to emit the irradiation beams (12, 12') in a predetermined number of irradiation directions (dᵢ) such that segments (gⱼ) of the micro-environment (M) remain un-irradiated between said irradiation beams (12, 12'), which segments (gⱼ) cover at least 50% of the micro-environment (M).

5. The system according to claim 4, wherein said predetermined number of irradiation directions (dᵢ) is two to four.

6. The system according to any one of claims 1 to 5, wherein the irradiation device (109) comprises an aperture (15), and wherein the controller (108) is further configured to set the aperture (15) of the irradiation device (109) for each irradiation direction (dᵢ) separately to match a contour (C) of the hypoxic region (H) of the tumor (3) as seen in that irradiation direction (dᵢ) and to control the irradiation device (109) to emit the irradiation beams (12) in that irradiation direction (dᵢ) through the aperture (15) such that the aperture (15) blocks the irradiation beams (12) from irradiating the normoxic region (N) and the micro-environment (M) during the treatment.

7. The system according to any one of claims 1 to 5, wherein the irradiation device (109) is configured to emit the irradiation beams (12') in different irradiation directions (dᵢ) that intersect at a single movable irradiation spot (R), and wherein the controller (108) is further configured to define a plurality of different target points (Tₖ) inside the hypoxic region (H) and no target points (Tₖ) outside the hypoxic region (H) and to move the irradiation spot (R) onto each of the defined target points (Tₖ) such that the irradiation spot (R) is neither moved to the normoxic region (N) nor to the micro-environment (M) during the treatment.

8. The system according to any one of claims 1 to 7, wherein the scanner (105) is configured to use a positron emission tomography to generate the scan (S').

9. The system according to claim 8, wherein the processor (106) is configured to calculate a standardized uptake value of the positron emission tomography scan and to determine the hypoxic region (H) as a region in which the standardized uptake value is equal to or smaller than 3.

10. The system according to claim 8 or 9, wherein the scanner (105) is configured to use a tracer 18F-FMISO or 18F-FDG for positron emission tomography.

11. The system according to any one of claims 8 to 10, wherein the scanner (105) is configured to use a computed tomography to generate the scan (S') by combining a result of the positron emission tomography with a result of the computed tomography.
